# FASCICULE DE BREVET EUROPEEN

(11) **EP 2 596 777 B1**
(45) Date de publication et mention de la délivrance du brevet: **18.02.2015**
(21) Numéro de dépôt: 12193864.1
(22) Date de dépôt: 22.11.2012
(51) Int. Cl.: A61F 13/00, A61F 13/08

(54) **Orthèse adaptée de compression/contention, pour le renforcement de la pompe musculo-aponévrotique du mollet**
Maßgeschneiderte Kompressions-/Stützorthese zur Verstärkung der Muskelfaszienpumpe der Wade
Adapted compression/containment orthosis for reinforcing the muscular-aponeurotic pump of the calf

(30) Priorité: 22.11.2011 FR 1160643
(43) Date de publication de la demande: 29.05.2013
(73) Titulaire: Innothera Topic International, 94110 Arcueil (FR)
(72) Inventeur: Cros, François, 94200 IVRY/SEINE (FR); Thiney, Grégory, 92600 ASNIERES/SEINE (FR)
(74) Mandataire: Dupuis-Latour, Dominique

(56) Documents cités:
- EP-A1- 1 656 916
- EP-A2- 1 240 880
- WO-A1-2006/134875
- WO-A1-2011/023650
- WO-A2-2011/143489
- FR-A1- 2 606 629
- FR-A1- 2 824 471

## Description

L'invention concerne les orthèses de compression veineuse élastique (CVE), qui sont indiquées dans les diverses manifestations cliniques d'insuffisance veineuse des membres inférieurs.

Ces orthèses, anciennement connues sous la dénomination de "bas (ou chaussettes) de contention" ou "collants de contention", sont des dispositifs médicaux textiles produisant un effet thérapeutique par compression des membres inférieurs, par opposition aux "bas de maintien" (ou encore "bas de soutien" ou "bas anti-fatigue") et aux "bas mode", qui ne sont pas des dispositifs médicaux à visée thérapeutique.

Les orthèses de CVE sont conçues pour produire un effet thérapeutique par compression du membre inférieur sur une étendue plus ou moins grande, habituellement avec un profil dégressif vers le haut à partir de la cheville. Selon le type d'orthèse, la pression mesurée à la cheville peut varier de 10 à plus de 36 mmHg (soit 13 à 48 hPa, le mmHg étant toutefois d'usage courant comme unité de mesure de pression dans le domaine de la phlébologie et de la compression médicale). Pour la France, les bas sont répartis selon le référentiel ASQUAL en quatre classes textiles, à savoir la classe I (13 à 20 hPa ≈ 10 à 15 mmHg à la cheville), la classe II (20 à 27 hPa ≈ 15 à 20 mmHg), la classe III (27 à 48 hPa ≈ 20 à 36 mmHg) et la classe IV (> 48 ha ≈ > 36 mmHg). Ces classes de compression peuvent être différentes pour d'autres pays.

Pour permettre une compression forte des membres inférieurs, ces orthèses sont réalisées à partir d'une maille tricotée de texture plus ou moins serrée avec incorporation d'un fil de trame élastique, généralement un élasthanne guipé.

Plus précisément, sous l'effet de la mise en place sur le membre, le textile tendu de l'orthèse exerce une compression résultant de la force de rappel des fibres élastiques qui composent le matériau, et l'application de ces forces de rappel élastique sur le périmètre du contour engendre en un point donné, selon la loi de Laplace, une pression locale inversement proportionnelle au rayon de courbure du contour en ce point.

Cette pression est la "pression textile" telle que définie et calculée au sens de la norme française NF G 30-102, partie B. On désignera dans la présente description par "pression" la moyenne des pressions localement exercées à une altitude donnée le long d'un contour de la jambe.

La maille et les fils, ainsi que le dimensionnement des rangées de mailles, sont choisis de manière à appliquer des pressions prédéterminées à différentes altitudes du membre inférieur, par exemple à la hauteur de la cheville, au départ du mollet, au niveau du mollet, au creux poplité, etc. jusqu'en haut de cuisse, altitudes notées conventionnellement *B*, *C* ... *G*. Ces différentes pressions sont définies pour chaque classe en référence à des gabarits métrologiques tels que la jambe-modèle de la norme française NF G 30-102 partie B, annexe B, correspondant à la jambe-modèle "type Hohenstein" selon le référentiel allemand RAL-GZ 387, ou tels que définis dans la prénorme européenne XP ENV 12718:2001

La caractéristique évoquée plus haut de dégressivité du profil de pression consiste à exercer une pression maximale à la cheville puis dégressive de la cheville au mollet ou à la cuisse. Elle repose sur le fait qu'en situation orthostatique la pression intraveineuse est dégressive de la cheville au mollet puis jusqu'à la cuisse. Il est donc logique d'appliquer une contre-pression correspondante, donc dégressive, afin de réduire les calibres veineux en proportion et induire un effet anti-stase.

En situation dynamique, comme lors de la marche, la situation est différente sur le plan physiologique, le mollet étant l'élément-clef de l'hémodynamique veineuse des membres inférieurs.

On a décrit notamment l'importance de l'effet de la "pompe musculaire" ou "pompe musculo-aponévrotique du mollet" (PMAM) en termes de flux sanguin veineux de retour, où les cycles physiologiques de contractions et de relâchements des muscles du mollet provoquent, par le jeu des ouvertures et fermetures des valvules veineuses, les vidanges et remplissages du réseau veineux du membre inférieur, qui se traduit par une baisse de la pression veineuse au niveau de la cheville. Le rendement de la PMAM se réduit progressivement avec l'âge des sujets, ce qui s'accompagne d'une hyperpression veineuse résiduelle qui aggrave naturellement les insuffisances veineuses chroniques.

L'insuffisance veineuse chronique est donc caractérisée par une défaillance de cet effet de pompe musculaire qui, de ce fait, joue un rôle majeur dans la genèse des troubles trophiques comme les ulcères.

Le point de départ de l'invention est la recherche d'un moyen permettant d'améliorer le rendement de la PMAM, voire de la suppléer, grâce à une orthèse compressive mieux adaptée à ce rôle que les orthèses qui ont pu être proposées jusqu'à présent et qui sont dégressives, car elles reposent sur l'analyse des pressions veineuses issues des situations orthostatiques.

Or l'étude de la physiologie veineuse, notamment par les outils récents de modélisation et de simulation de contention tels que ceux décrits dans le WO 2006/087442 A1 (Laboratoires Innothéra), montre que l'efficacité d'une orthèse de CVE réside plutôt dans l'amélioration du rendement de la PMAM, dès lors que l'on est capable de faire fonctionner celle-ci.

Le FR 2 824 471 B1 (Rodier) décrit une approche consistant à réaliser une "compression/contention élective" au moyen d'un bas multizone à tricotage différencié, associant une région en maille très élastique au niveau du pied et de la cheville, suivie par une région en maille peu élastique depuis le bas du mollet jusqu'au creux poplité, et prolongée par une région en maille à nouveau très élastique depuis le genou jusqu'en haut de cuisse. L'idée de base consiste à prévoir des zones à effet plutôt compressif (pied, cheville et cuisse) de part et d'autre d'une zone à effet plutôt contentif (mollet). Cette dernière zone de l'orthèse produira moins d'effet au repos que celles qui l'entourent, en revanche lors des contractions du muscle du mollet elle exercera une compression accrue, augmentant la puissance et renforçant l'effet de vidange de la PMAM.

Il convient de préciser à cet égard que les termes de "compression" et de "contention" définissent des effets bien différents, bien qu'ils soient parfois confondus dans le langage commun :
- la "compression" est l'effet produit par une orthèse élastique, aussi bien au repos qu'à l'effort, sur un segment de membre du fait des forces plus ou moins puissantes de rappel des fibres élastiques de cette orthèse. Ces forces agissent de manière quasi constante sur le membre : au repos, la compression est présente à la valeur de pression nominale, et à l'effort l'effet de cette compression est faiblement augmenté par la contraction des masses musculaires ;
- à l'inverse, la "contention" est l'effet produit par une orthèse qui agit de manière différenciée (effort/repos) sur un segment de membre sous l'action d'une structure considérée comme inélastique (mais déformable), par exemple un bandage non élastique, également désigné "bandage à allongement court". Au repos, ce type de bande exerce une pression faible, voire nulle ; en revanche, pendant la contraction musculaire, elle s'oppose aux augmentations locales de volume du mollet qui vient buter sur la structure non élastique, la pression se trouvant ainsi fortement augmentée. La contention est donc efficace et active à l'ef fort, et quasi inactive au repos.

Il est d'usage dans la littérature scientifique sur le sujet de considérer qu'une orthèse est contentive, ou "rigide" lorsqu'elle produit une augmentation d'au moins 10 mmHg (13 hPa) par centimètre d'augmentation de la circonférence du membre au point situé au point de jonction entre le tendon d'Achille et les muscles du mollet. La "rigidité" est ici entendue au sens de la définition de la prénorme européenne XP ENV 12718:2001, c'est-à-dire l'"*augmentation de compression par centimètre d'augmentation de circonférence de la jambe, exprimée en hectopascals par centimètre et*/*ou en millimètres de mercure par centimètre".*

C'est pour désigner ces deux notions différentes que seront utilisés dans la suite les deux termes respectifs de "compression" (ou "compressif") et de "contention" (ou "contentif").

Au regard de ces définitions, la proposition du FR 2 824 471 B1 précité, qui ne met en oeuvre que des fils et des mailles plus ou moins élastiques sur la hauteur de l'orthèse, ne produit qu'un effet contentif très partiel au niveau du mollet.

D'autres orthèses constituées de zones toutes élastiques mais à élasticité différenciée sont proposées par les EP 0 934 043 B1 (Couzan) ou EP 1 240 880 A2 (Stolk). Ces deux documents enseignent de réaliser un bas ou une chaussette avec une zone moins rigide (plus élastique) dans la région du mollet, respectivement de façon uniforme sur toute la circonférence du mollet, ou bien seulement dans la région postérieure de celui-ci. Les orthèses décrites, qui sont dépourvues de toute structure inélastique, ne procurent donc aucun effet de "contention", au sens explicité plus haut, avec un effet de butée du mollet contre une structure non élastique, en cas d'augmentation locale du volume de celui-ci.

Il en est de même pour le produit divulgué par le WO 2006/134875 A1, qui prévoit des éléments discrets ajoutés à des endroits choisis d'une chaussette ou d'un bas "anti-fatigue", et destinés à augmenter la sensation de compression ressentie par le porteur, donc sans finalité ni fonction contentives. Au surplus, aucun de ces éléments ne s'étend sur la circonférence du mollet, et de ce fait ne peut pas engendrer de contention, faute de pouvoir créer un obstacle inélastique à l'augmentation de volume du mollet à l'effort.

En outre, du point de vue de la technologie, toutes ces structures "multizone" de l'art antérieur s'avèrent difficiles à réaliser en pratique, compte tenu de la difficulté qu'il y a à régler la machine de tricotage pour obtenir les profils d'élasticité variable requis, avec des transitions très abruptes entre des textures très hétérogènes correspondant aux différentes zones du bas ou de la chaussette.

D'autre part et surtout, ces orthèses que l'on pourrait qualifier de "semi-contentives" ne sont pas adaptées spécifiquement à un patient donné. Concrètement, le praticien se contente de sélectionner une orthèse dans une grille de tailles après avoir mesuré le périmètre de la cheville et du mollet. Ceci aboutit en pratique à une solution de compromis qui ne tient pas compte de la morphologie réelle du mollet, qui peut être très variable d'un patient à l'autre et qui ne peut être décrite convenablement par une unique mesure du périmètre maximal du mollet.

Cet inconvénient est particulièrement accru dans le cadre de produits censés produire un réel effet contentif (au sens défini plus haut), car le renforcement de l'effet de PMAM est subordonné à un ajustement précis de la structure non élastique au segment de membre concerné, sur toute l'étendue de celui-ci : si la structure non élastique n'est pas en contact étroit avec le membre au repos, elle ne procurera que très peu d'effet pour une augmentation faible ou modérée du volume du muscle ; si au contraire sa dimension est trop faible, elle exercera une contrainte sur le membre même au repos, avec des effets délétères sur la circulation sanguine, outre une sensation de carcan risquant de rendre le port de l'orthèse particulièrement inconfortable pour le patient.

Il apparait ainsi souhaitable de pouvoir réaliser des orthèses procurant un véritable effet contentif sur le mollet grâce à une structure non élastique (et non pas une structure à élasticité moindre), adaptée à la morphologie exacte du segment de membre de chaque patient.

La structure non élastique doit toutefois être déformable, à la différence par exemple du produit divulgué par le EP 1 656 916 A1, qui est une orthèse de contention orthopédique indéformable, destinée à former une attelle pour immobiliser un membre traumatisé : il ne s'agit pas d'une contention de même nature que celle de la présente invention, qui doit pouvoir être mise en oeuvre par un article, tel qu'un bas ou une chaussette, susceptible d'être enfilé et retiré à volonté par le patient, et qui une fois en place n'entrave pas les mouvements du membre enveloppé par l'orthèse.

Si l'on veut disposer d'un produit contentif rigide sur mesure, adapté spécifiquement au patient, une première solution consiste à utiliser des bandages multicouche, avec la difficulté bien connue qu'il y a à bien ajuster le bandage, ni trop serré (il entrainerait un écrasement du mollet) ni trop lâche (il ne produirait plus aucun effet), d'où un résultat très "opérateur-dépendant". Comme expliqué plus haut, l'ajustement d'un produit rigide de contention est très critique, à la différence d'une structure élastique compressive, beaucoup plus tolérante.

En outre, le bandage doit être refait régulièrement, à chaque fois avec le même soin pour un bon ajustement.

Le FR 2 912 644 (Mollard et al.) divulgue une telle technique, avec en outre superposition d'un élément compressif et d'un élément contentif. L'élément contentif est une bande d'un film ultramince perforé, par exemple de polyéthylène, conditionné en une bobine. Ce film est déroulé autour du membre de manière à envelopper celui-ci, puis un bas de contention est enfilé sur le bandage de manière à procurer l'effet compressif.

Mais outre l'habileté particulière nécessaire à la mise en place du bandage, une fois celui-ci mis en place il n'existe aucune possibilité de réajustement, sauf à reprendre la totalité du processus. Enfin, ce produit est à usage unique et ne permet pas un retrait temporaire de l'orthèse, par exemple le temps d'un examen ou du changement d'un pansement.

Pour ces raisons, les patients préfèrent généralement recourir à une autre solution, sous forme d'orthèse tricotée à enfiler, plus commode et plus esthétique.

Il s'agit alors de fabriquer un produit rigide sur mesure, parfaitement adapté à la morphologie particulière du patient. La technique consiste à prendre les mesures du mollet de la façon la plus complète, avec des cotes à plusieurs altitudes. L'orthèse est ensuite tricotée sur un métier à plat, puis mise en forme par réalisation d'une couture sur tout son long, ce qui nécessite une étape de confection supplémentaire. On comprendra que cette technique de sur-mesure intégral est longue à mettre en oeuvre, compliquée et donc couteuse et ne permet pas une grande diffusion des produits rigides de contention, malgré leurs avantages thérapeutiques évidents.

Le problème de l'invention est ainsi de pouvoir réaliser une orthèse de contention (produit rigide) qui puisse se présenter sous forme d'un produit final "sur mesure", donc parfaitement ajusté à la morphologie du patient, mais qui pour autant ne nécessite pas une fabrication par des techniques conventionnelles de "sur mesure", longues et couteuses.

On verra en particulier que l'invention peut être mise en oeuvre (i) par un métier circulaire (et non un métier à plat, qui nécessiterait une étape de confection supplémentaire pour la réalisation de la couture) (ii) réalisant un produit standard, donc avec possibilité de fabrication à un cout raisonnable et en grande série.

Et ceci avec une nouvelle structure d'orthèse de CVE :
- qui assure un renforcement des effets bénéfiques de la PMAM par une contention appropriée du mollet,
- qui soit technologiquement aisée à réaliser,
- et qui puisse être facilement adaptée à des morphologies de jambe très différentes rencontrées dans la population des patients concernés.

On verra également que l'invention permet d'obtenir une orthèse de CVE du membre inférieur qui applique au niveau du mollet non plus une compression plus ou moins renforcée, mais une véritable contention en plaçant autour du mollet un élément essentiellement rigide, c'est-à-dire non élastiquement déformable. En outre, à cette rigidité forte au mollet (effet de contention) sera associée une rigidité faible à la cheville (effet de compression).

En effet, une rigidité forte au mollet est considérée comme un moyen d'optimisation de la PMAM, qui est le moteur principal du retour veineux dans les membres inférieurs. Mais la rigidité forte au mollet doit être associée à une rigidité faible (donc une déformabilité élevée) en cheville pour garantir une facilité de mise en place, de retrait et de tolérance du produit - notamment pour éviter une compression trop forte, qui serait rapidement intolérable notamment pour un patient alité ou inactif.

Une solution à ce problème est décrite par la Demanderesse dans le EP 2 452 658 A1 (publié le 16 mai 2012, donc postérieurement à la date de priorité de la présente demande). Cette solution consiste à incorporer lors de la fabrication un fil thermoformable à la trame de l'orthèse dans la région du mollet. L'orthèse est ensuite mise en place sur une forme et chauffée localement pour la rendre inextensible, donc contentive, dans cette région sous l'effet des changements des caractéristiques mécaniques du fil thermoformable.

Cette solution requiert toutefois une modification du processus de tricotage pour pouvoir incorporer le fil thermoformable lors de la fabrication de l'orthèse.

La présente invention à pour but de présenter une alternative à cette solution, qui ne présente pas cet inconvénient et puisse être mise en oeuvre à partir d'une orthèse de structure conventionnelle et sans modification du processus de tricotage.

Essentiellement, l'idée de base de l'invention consiste à réaliser une orthèse compressive par des techniques conventionnelles, mais en intégrant au produit une partie contentive réalisée par application puis séchage d'une résine biocompatible appropriée, permettant d'obtenir un durcissement du textile dans la région où cette résine a été appliquée.

Si cette opération est réalisée avec l'orthèse enfilée sur la jambe du patient - ou, de préférence, sur un gabarit représentatif de la morphologie de cette jambe, pour éviter au patient les désagréments de cette opération -, et si l'application de la résine est soigneusement localisée à la région du mollet, on disposera alors *in fine* d'un produit compressif/contentif parfaitement adapté à la forme du mollet du patient.

Le produit ainsi obtenu permet donc d'appliquer au mollet une contention efficace, grâce à la partie contentive dont la forme sera personnalisée en fonction du patient, cette région étant en quelque sorte "moulée en place" sur le mollet du patient. Cette partie contentive dans la région du mollet sera associée à une partie compressive conventionnelle sur le reste de la jambe, tout particulièrement dans la région de la cheville.

Plus précisément, l'invention propose une orthèse de CVE de même finalité que le FR 2 824 471 B1 précité, c'est-à-dire une orthèse de compression médicale en forme de chaussette, de bas ou de collant destinée à agir spécifiquement sur la PMAM.

Une telle orthèse d'après la revendication 1 comporte, de manière en elle-même connue, (i) une partie distale compressive élastique, apte à couvrir la cheville en s'étendant jusqu'avant le début du mollet, au niveau du point de jonction entre le tendon d'Achille et les muscles du mollet, point généralement dénommé B1, et (ii) une partie proximale contentive, prolongeant la partie distale compressive et attenante à celle-ci, et enveloppant sur sa circonférence une région du mollet comprise entre le niveau du point de jonction entre le tendon d'Achille et les muscles du mollet et le niveau situé en dessous de la tubérosité tibiale.

La partie distale est réalisée par tricotage d'un fil de tricot et d'un fil de trame, le dimensionnement et la nature des fils de tricot et de trame ainsi que la structure de maille étant choisis de manière à exercer en direction circonférentielle, après que l'orthèse a été mise en place sur le membre, une force de rappel élastique propre à produire une compression du membre à un niveau de pression thérapeutique désirée. La partie proximale contentive est quant à elle une partie tubulaire déformable tricotée en continuité avec la partie distale compressive élastique.

De façon caractéristique de l'invention, la partie proximale contentive est essentiellement non élastique, et elle incorpore une résine biocompatible durcie, par exemple une résine acrylique monocomposant durcissable par évaporation.

Le fil de tricot peut notamment être un élasthanne guipé polyamide et/ou coton, et le fil de trame un élasthanne guipé polyamide et/ou coton.

La partie proximale peut être une partie présentant au niveau de la circonférence maximale du mollet une forte rigidité, de 15 ± 2 mmHg/cm (≈ 20 ± 2 hPa/cm), ou une rigidité modérée, de 5 ± 2 mmHg/cm (≈ 7 ± 2 hPa/cm).

La partie distale compressive élastique peut être une partie faiblement compressive apte à exercer une pression de 10 à 20 mmHg (13 à 27 hPa), ou bien modérément compressive apte à exercer une pression de 20 à 30 mmHg (27 à 40 hPa), au niveau de la circonférence minimale de la cheville.

L'invention vise également un procédé spécifique de mise à la mesure de la jambe d'un patient d'une orthèse de compression/contention médicale du membre inférieur.

Ce procédé d'après la revendication 8 comprend les étapes suivantes : obtention d'une orthèse telle que ci-dessus dans un état initial brut de fabrication, sans résine biocompatible ; mise en place de l'orthèse sur un gabarit représentatif de la morphologie du mollet du patient ; application in *situ* sur l'orthèse, dans la région de la partie proximale contentive, d'une résine biocompatible durcissable ; durcissement de la résine avec l'orthèse maintenue sur le gabarit ; et retrait de l'orthèse dans son état fini. L'orthèse présente alors une partie proximale contentive rendue rigide suite au durcissement de la résine et conservant les dimensions correspondantes du mollet du patient, permettant à cette partie proximale contentive de venir épouser parfaitement la forme du mollet.

On va maintenant décrire un exemple de mise en oeuvre de l'invention, en référence aux dessins annexés où les mêmes références numériques désignent d'une figure à l'autre des éléments identiques ou fonctionnellement semblables.
La Figure 1 est une vue d'ensemble d'une orthèse selon l'invention, à l'état libre.
La Figure 2 est une vue en élévation de cette même orthèse enfilée sur un membre, avec indication des diverses altitudes normalisées auxquelles sont mesurées les pressions appliquées par l'orthèse sur le membre.
La Figure 3 illustre les étapes successives du procédé de mise en oeuvre selon l'invention, destiné à mettre l'orthèse à la mesure de la jambe du patient.

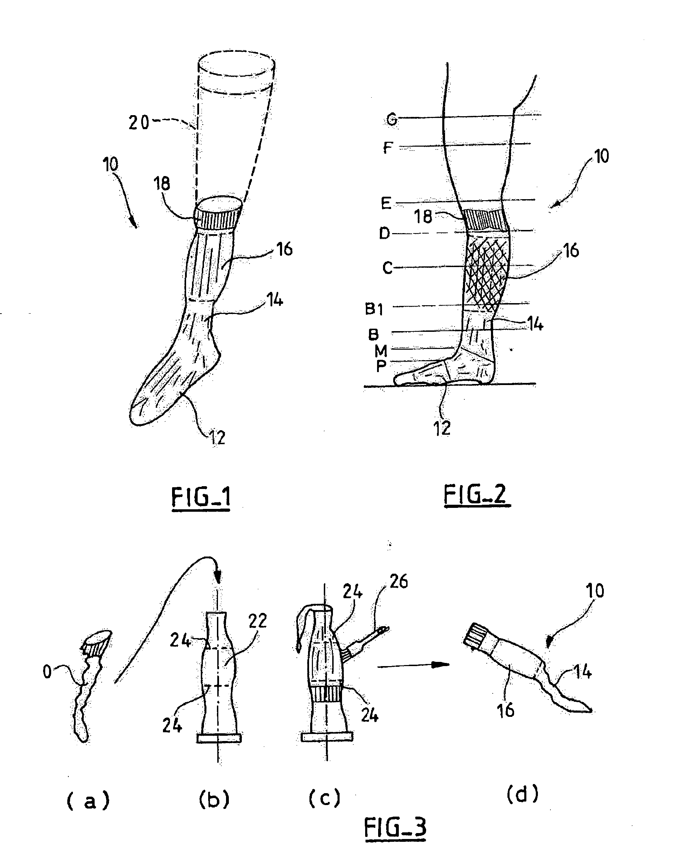

Sur les Figures 1 et 2, la référence 10 désigne de façon générale l'orthèse de l'invention, qui est une orthèse tricotée réalisée selon des procédés conventionnels sur un métier circulaire. Cette orthèse 10 de forme tubulaire comprend une partie 12 enveloppant le pied et une partie de jambe avec une partie distale 14 enveloppant la cheville et une partie proximale 16 enveloppant le mollet. L'ensemble s'étend jusqu'à un niveau situé au-dessous du genou, dans le cas où l'orthèse est une chaussette "mi-bas" (ou "bas-jarret"). Dans ce dernier cas, l'orthèse est terminée par une partie tricotée terminale de type "bord-côtes" 18.

Cette configuration en forme de chaussette n'est pas limitative, et l'invention peut être également réalisée sous forme d'un "bas-cuisse", prolongé par une partie de cuisse compressive 20. L'orthèse de l'invention peut être également réalisée sous forme d'un collant, et/ou dépourvue de partie de pied 12 (bas ou collant de type "pied ouvert").

Les différentes parties attenantes de l'orthèse que l'on vient de décrire sont tricotées en continu sur le métier circulaire, c'est-à-dire que la réalisation de cette orthèse ne requiert aucune étape de confection pour l'assemblage de pièces distinctes, à l'exception bien entendu des opérations de couture de la pointe au niveau de la partie de pied 12, si cette dernière est présente.

Sur la Figure 2, on a représenté les diverses altitudes du membre inférieur telles que définies par le référentiel morphologique indiqué en introduction (jambe-modèle "type Hohenstein") utilisant les notations normalisées :
B : cheville, au point de sa circonférence minimale ;
B1 : point de jonction entre le tendon d'Achille et les muscles du mollet ;
C : mollet, au point de sa circonférence maximale ;
D : juste au-dessous de la tubérosité tibiale (c'est-à-dire juste au-dessous du genou) ;
E : au centre de la rotule et au-dessus de l'arrière du genou (c'est-à-dire au niveau du creux poplité) ;
F : en milieu de cuisse ; et
G : en haut de cuisse.

Le mollet est le segment de membre compris entre les niveaux B1 et D, et la cheville est le segment de membre situé au-dessous du niveau B1.

La pression exercée à l'altitude B (au périmètre minimal de la cheville) est la pression prescrite pour la classe normalisée choisie (I, II, III ou IV).

Les valeurs de pression peuvent être relevées par exemple sur dynamomètre conformément à la norme précitée NF G 30-102 partie B, après avoir enfilé l'orthèse sur un gabarit de référence tel que le modèle de jambe Hohenstein prescrit par cette norme.

La pression exercée au niveau de la cheville au point de sa circonférence minimale (niveau B) par la partie distale compressive élastique 14 doit être une pression thérapeutique efficace. Les valeurs suivantes peuvent être retenues, en fonction des besoins du patient :
- 10 à 20 mmHg (13 à 27 hPa) pour une compression relativement faible de la cheville ;
- 20 à 30 mmHg (27 à 40 hPa) pour une compression modérée de la cheville.

La partie distale compressive élastique 14 produisant ces pressions thérapeutiques est réalisée à partir d'une maille tricotée de texture plus ou moins serrée avec incorporation d'un fil de trame élastique, par exemple en utilisant :
- comme fil de trame, un fil tel qu'un élasthanne ou un mélange d'élasthanne et d'élasto-diène (latex de caoutchouc synthétique), guipé polyamide et/ou coton ; et
- comme fil de tricot (fil de maille), également un élasthanne guipé polyamide et/ou coton, de préférence avec un titre (masse linéique) moindre que celui du fil de trame.

De façon caractéristique de l'invention, la partie proximale 16 est une partie contentive (c'est-à-dire essentiellement non élastique dans l'état final de l'orthèse), de forme tubulaire, s'étendant :
- en direction verticale : sur l'étendue du mollet, c'est-à-dire sur la région comprise entre le niveau B1 (jonction entre le tendon d'Achille et les muscles du mollet) et le niveau D (au-dessous du genou), ou tout au moins sur la majeure partie de cette région ; on notera que la cheville (région s'étendant autour du niveau B) ne fait jamais partie de cette région recouverte par la partie proximale 16 ; et
- en direction circonférentielle : sur toute la circonférence du mollet.

Cette partie non élastique est réalisée sur mesure, de la manière que l'on va exposer ci-dessous, c'est-à-dire qu'elle présente une configuration extérieure précisément ajustée à la forme et aux dimensions du mollet du patient. De la sorte, une fois l'orthèse enfilée sur le membre cette partie exercera l'effet de contention recherché, c'est-à-dire qu'au repos elle n'exercera essentiellement aucun effort de contention, mais qu'à l'effort elle opposera au membre une rigidité procurant l'effet contentif au niveau d'efficacité souhaité.

En ce qui concerne la rigidité R_{c} de cette partie proximale contentive 16, on peut retenir les valeurs suivantes (selon la prénorme européenne XP ENV 12718:2001 précitée) :
- pour une contention forte : R_{c} = 15 ± 2 mmHg/cm (≈ 20 ± 2 hPa/cm) ;
- pour une contention modérée : R_{c} = 5 mmHg/cm (≈ 7 hPa/cm).

Ces valeurs R_{c} sont mesurées à l'altitude C, c'est-à-dire au point de la circonférence maximale du mollet.

En jouant séparément, d'une part sur l'élasticité de la partie distale compressive 14 au niveau de la cheville, et d'autre part sur la rigidité de la partie proximale contentive 16 au niveau du mollet, il est possible de combiner plusieurs effets de compression/contention, par exemple :
- faible compression en cheville/forte contention au mollet ;
- compression modérée en cheville/forte contention au mollet ;
- faible compression en cheville/contention modérée au mollet ; ou
- compression modérée en cheville/contention modérée au mollet.

Très avantageusement, les deux parties distale 14 et proximale 16 sont tricotées en continu au cours d'une même séquence sur la machine de tricotage, ce qui évite toute étape de confection pour l'assemblage de parties rapportées. On peut ainsi tricoter la partie proximale 16 avec les mêmes types de fils que la partie distale compressive élastique 14, à savoir :
- comme fil de trame, un fil d'élasthanne guipé polyamide et/ou coton, et
- comme fil de tricot, un fil d'élasthanne guipé polyamide et/ou coton de moindre titre.

Le produit peut être tricoté suivant des techniques usuelles sur un métier circulaire classique, tel qu'un métier *Santoni.*

De façon caractéristique de l'invention, la partie proximale contentive non élastique 16 est obtenue par ajout d'une résine.

Cette opération est réalisée de la manière illustrée Figure 3.

L'orthèse 10 qui vient d'être tricotée de la manière conventionnelle se présente initialement sous la forme d'un produit standard, c'est-à-dire un produit qui n'est pas sur mesure (étape a) ; il est seulement prévu, comme pour les orthèses de CVE classiques, et même pour tout article d'habillement, des tailles standards appropriées, à choisir dans une grille dimensionnelle.

Cette orthèse est alors placée (étape b) sur un gabarit 22 correspondant à la morphologie du patient dans la région du mollet. Cette région peut être notamment délimitée par des repères tels que 24, visibles par transparence une fois l'orthèse enfilée.

L'étape suivante (étape c) consiste à ajouter dans la région du mollet, c'est-à-dire entre les repères 24, une résine biocompatible, par exemple par application au moyen d'un pinceau 26, par pulvérisation contrôlée ou encore par trempage.

Un exemple de résine utilisable à cet effet est par exemple la résine *Plastidurex,* qui est une résine acrylique monocomposant commercialisée par REAL Composites, et qui est utilisée par exemple dans le domaine de la décoration pour la rigidification de papiers et de tissus, la création d'abat-jours, etc.

Une autre résine utilisable est la résine *SILDOC RTV AD35,* qui est une résine silicone bicomposant, également commercialisée par REAL Composites, et qui est utilisée par exemple dans le domaine des moulages corporels.

Cette résine est appliquée jusqu'à saturation du textile, puis on attend son séchage, par évaporation.

Dans un exemple de mise en oeuvre, on a constaté qu'après durcissement par évaporation, 12 g de résine avaient été ajoutés à la chaussette par rapport au poids initial de celle-ci, pour une application dans la région du mollet telle que définie plus haut, sur toute la circonférence de la chaussette. La rigidité finale obtenue était de 15 mm Hg/cm (20 hPa/cm) environ.

L'orthèse peut alors être retirée de la forme (étape d). Elle aura alors pris sa forme définitive, "sur mesure", avec une partie proximale contentive 16 devenue rigide et ayant pris une forme épousant parfaitement le galbe et les dimensions du mollet du patient, et une partie distale compressive élastique 14, de sorte que l'on se trouve alors en présence d'un produit associant une rigidité forte au mollet (partie proximale contentive 16) et une rigidité faible à la cheville (partie distale compressive élastique 14) assurant une compression du membre inférieur à un niveau thérapeutique.

## Revendications

1. Une orthèse de compression/contention médicale du membre inférieur en forme de chaussette, de bas ou de collant,
cette orthèse (10) comportant :
- une partie distale compressive (14) élastique, apte à couvrir la cheville en s'étendant jusqu'avant le début du mollet, au niveau du point de jonction entre le tendon d'Achille et les muscles du mollet,
cette partie distale étant réalisée par tricotage d'un fil de tricot et d'un fil de trame, le dimensionnement et la nature des fils de tricot et de trame ainsi que la structure de maille étant choisis de manière à exercer en direction circonférentielle, après que l'orthèse a été mise en place sur le membre, une force de rappel élastique propre à produire une compression du membre à un niveau de pression thérapeutique désirée ; et
- une partie proximale contentive (16), prolongeant la partie distale compressive et attenante à celle-ci, et enveloppant sur toute sa circonférence une région du mollet comprise, en direction verticale, entre le niveau (B1) du point de jonction entre le tendon d'Achille et les muscles du mollet et le niveau (D) situé en dessous de la tubérosité tibiale ; cette partie proximale contentive étant une partie tubulaire déformable tricotée en continuité avec la partie distale compressive élastique,
cette orthèse étant **caractérisée en ce que** la partie proximale contentive :
- est essentiellement non élastique, et
- incorpore une résine biocompatible durcie.

2. L'orthèse de la revendication 1, dans laquelle la résine biocompatible est une résine acrylique monocomposant durcissable par évaporation.

3. L'orthèse de la revendication 1, dans laquelle le fil de tricot et/ou le fil de trame est un élasthanne guipé polyamide et/ou coton.

4. L'orthèse de la revendication 1, dans laquelle la partie proximale contentive est une partie présentant au niveau de la circonférence maximale du mollet une forte rigidité, de 15 ± 2 mmHg/cm (≈20 ± 2 hPa/cm).

5. L'orthèse de la revendication 1, dans laquelle la partie proximale contentive est une partie présentant au niveau de la circonférence maximale du mollet une rigidité modérée, de 5 ± 2 mmHg/cm (≈ 7 ± 2 hPa/cm).

6. L'orthèse de la revendication 1, dans laquelle la partie distale compressive élastique est une partie faiblement compressive, apte à exercer une pression de 10 à 20 mmHg (13 à 27 hPa) au niveau de la circonférence minimale de la cheville.

7. L'orthèse de la revendication 1, dans laquelle la partie distale compressive élastique est une partie modérément compressive, apte à exercer une pression de 20 à 30 mmHg (27 à 40 hPa) au niveau de la circonférence minimale de la cheville.

8. Un procédé de mise à la mesure de la jambe d'un patient d'une orthèse de compression/contention médicale du membre inférieur, ce procédé comprenant les étapes suivantes :
- obtention d'une orthèse (10) selon l'une des revendications 1 à 7 dans un état initial brut de fabrication, sans résine biocompatible ;
- mise en place de l'orthèse sur un gabarit représentatif de la morphologie du mollet du patient ;
- application *in situ* sur l'orthèse, sur toute la circonférence de la région de la partie proximale contentive, d'une résine biocompatible durcissable ;
- durcissement de la résine avec l'orthèse maintenue sur le gabarit ; et
- retrait de l'orthèse dans son état fini,
l'orthèse dans son état fini présentant une partie proximale contentive (16) rendue rigide suite audit durcissement de la résine et conservant les dimensions correspondantes du mollet du patient, permettant à cette partie proximale contentive de venir épouser parfaitement la forme du mollet.

## Patentansprüche

1. Medizinische Kompressions-/Stützorthese der unteren Extremität in Form eines Kniestrumpfes, eines Strumpfes oder einer Strumpfhose,
wobei diese Orthese (10) Folgendes aufweist:
- einen elastischen distalen Kompressionsteil (14), der geeignet ist, den Knöchel zu bedecken, wobei er sich bis vor den Beginn der Wade auf der Höhe der Verbindungsstelle zwischen der Achillessehne und den Muskeln der Wade erstreckt,
wobei dieser distale Teil durch Stricken eines Strickfadens und eines Schussfadens hergestellt ist, wobei die Dimensionierung und die Beschaffenheit der Strick- und Schussfäden sowie die Maschenstruktur derart gewählt sind, dass in Umfangsrichtung, nachdem die Orthese auf der Extremität angebracht worden ist, eine elastische Rückholkraft ausgeübt wird, die geeignet ist, eine Kompression der Extremität mit einem Niveau entsprechend dem gewünschten therapeutischen Druck zu erzeugen; und
- einen proximalen Stützteil (16), der den distalen Kompressionsteil verlängert und an diesen angrenzt und auf seinem gesamten Umfang einen Bereich der Wade umhüllt, der in vertikaler Richtung zwischen der Höhe (B1) der Verbindungsstelle zwischen der Achillessehne und den Muskeln der Wade und der Höhe (D), die sich unterhalb des Schienbeinhöckers befindet, liegt;
wobei dieser proximale Stützteil ein verformbarer rohrförmiger Teil ist, der in Kontinuität mit dem elastischen distalen Kompressionsteil gestrickt ist,
wobei diese Orthese **dadurch gekennzeichnet ist, dass** der proximale Stützteil:
- im Wesentlichen nicht elastisch ist, und
- ein ausgehärtetes biokompatibles Harz enthält.

2. Orthese nach Anspruch 1, bei der das biokompatible Harz ein durch Abdampfen aushärtbares Einkomponentenacrylharz ist.

3. Orthese nach Anspruch 1, wobei der Strickfaden und/oder der Schussfaden ein mit Polyamid und/oder Baumwolle umsponnener Elastanfaden ist.

4. Orthese nach Anspruch 1, wobei der proximale Stützteil ein Teil ist, der auf der Höhe des maximalen Umfangs der Wade eine hohe Steifigkeit aufweist, von 15 ± 2 mmHg/cm (≈ 20 ± 2 hPa/cm).

5. Orthese nach Anspruch 1, wobei der proximale Stützteil ein Teil ist, der auf der Höhe des maximalen Umfangs der Wade eine mäßige Steifigkeit aufweist, von 5 ± 2 mmHg/cm (≈ 7 ± 2 hPa/cm).

6. Orthese nach Anspruch 1, wobei der elastische distale Kompressionsteil ein schwach kompressiver Teil ist, der geeignet ist, einen Druck von 10 bis 20 mmHg (13 bis 27 hPa) auf der Höhe des minimalen Umfangs des Knöchels auszuüben.

7. Orthese nach Anspruch 1, wobei der elastische distale Kompressionsteil ein mäßig kompressiver Teil ist, der geeignet ist, einen Druck von 20 bis 30 mmHg (27 bis 40 hPa) auf der Höhe des minimalen Umfangs des Knöchels auszuüben.

8. Verfahren zur maßgeschneiderten Anpassung einer medizinischen Kompressions-/Stützorthese der unteren Extremität an das Bein eines Patienten, wobei dieses Verfahren die folgenden Schritte aufweist:
- Beschaffen einer Orthese (10) nach einem der Ansprüche 1 bis 7 in einem dem Herstellungszustand entsprechenden Anfangszustand ohne biokompatibles Harz;
- Platzieren der Orthese auf eine die Form der Wade des Patienten darstellende Schablone;
- Aufbringen eines aushärtbaren biokompatiblen Harzes in situ auf die Orthese über den gesamten Umfang des Bereichs des proximalen Stützteils;
- Aushärten des Harzes mit der auf der Schablone gehaltenen Orthese und
- Abziehen der Orthese in ihrem Endzustand,
wobei die Orthese in ihrem Endzustand einen proximalen Stützteil (16) aufweist, der nach der Aushärtung des Harzes steif geworden ist und die entsprechenden Abmessungen der Wade des Patienten behalten hat, was es diesem proximalen Stützteil ermöglicht, sich perfekt an die Form der Wade anzuschmiegen.

## Claims

1. An orthosis for medical compression/splinting of the lower limb, in the form of a sock, a stocking or a pair of tights,
said orthosis (10) including:
- an elastic compressive distal portion (14), adapted to cover the ankle, extending to before the beginning of the calf, at the point where the Achilles tendon joins the calf muscles,
said distal portion being made by knitting a knit yarn and a weft yarn, the dimensioning and the nature of the knit and weft yarns as well as the knit structure being chosen in such a way to exert in the circumference direction, once the orthosis has been placed on the limb, an elastic return force likely to produce a compression of the limb at a desired therapeutic level of pressure; and
- a splint proximal portion (16), continuing the compressive distal portion and adjacent thereto, and enveloping, over the whole periphery thereof, a region of the calf comprised, in the vertical direction, between the level (B1) of the point where the Achilles tendon joins the calf muscles and the level (D) located below the tibial tuberosity;
said splint proximal portion being a deformable tubular portion knitted in continuation with the elastic compressive distal portion,
said orthosis being **characterized in that** the splint proximal portion:
- is essentially non-elastic, and
- incorporates a hardened biocompatible resin.

2. The orthosis of claim 1, wherein the biocompatible resin is an evaporation-hardenable single-component acrylic resin.

3. The orthosis of claim 1, wherein the knit yarn and/or the weft yarn is a polyamide and/or cotton covered spandex yarn.

4. The orthosis of claim 1, wherein the splint proximal portion is a portion having, at the level of the calf maximum circumference, a high rigidity, of 15 ± 2 mmHg/cm (≈ 20 ± 2 hPa/cm).

5. The orthosis of claim 1, wherein the splint proximal portion is a portion having, at the level of the calf maximum circumference, a moderate rigidity, of 5 ± 2 mmHg/cm (≈ 7 ± 2 hPa/cm).

6. The orthosis of claim 1, wherein the elastic compressive distal portion is a low compression portion, adapted to exert a pressure of 10 to 20 mmHg (13 to 27 hPa) at the level of the ankle minimum circumference.

7. The orthosis of claim 1, wherein the elastic compressive distal portion is a moderate compression portion, adapted to exert a pressure of 20 to 30 mmHg (27 to 40 hPa) at the level of the ankle minimum circumference.

8. A method for tailoring an orthosis for medical compression/splinting of the lower limb to the size of a patient's leg, said method comprising the following steps:
- obtaining an orthosis (10) according to one of claims 1 to 7, in a rough manufactured initial state, with no biocompatible resin;
- placing the orthosis onto a template representative of the morphology of the patient's calf;
- applying *in situ* on the orthosis, over the whole circumference of the region of the splint proximal portion, a hardenable biocompatible resin;
- hardening the resin with the orthosis maintained on the template; and
- removing the orthosis in its finished state,
the orthosis in its finished state having a splint proximal portion (16) made rigid following said hardening of the resin and keeping the corresponding dimensions of the patient's calf, which allows this splint proximal portion to perfectly fit the shape of the calf.
